# EUROPÄISCHE PATENTANMELDUNG

(11) **EP 3 865 496 A1**
(43) Veröffentlichungstag der Anmeldung: **18.08.2021**
(21) Anmeldenummer: 20156820.1
(22) Anmeldetag: 12.02.2020
(51) Int. Cl.: C07H 1/00, C07H 1/06, C07H 3/02

(54) **VERFAHREN ZUR HERSTELLUNG FARBLOSER KOHLENHYDRATE**

(71) Anmelder: Savanna Ingredients GmbH, 50189 Elsdorf (DE)
(72) Erfinder: JUNKLEWITZ, Anna, 42119 Wuppertal (DE); MOHR, Stephan, 53879 Euskirchen (DE); BUTZ, Steffen, 52372 Kreuzau (DE); KOCH, Timo, 50189 Elsdorf (DE)
(74) Vertreter: Fabry, Bernd

(57) **Zusammenfassung**

Vorgeschlagen wird ein Verfahren zur Herstellung farbloser Kohlenhydrate, umfassend oder bestehend aus den folgenden Schritten:
(a) Bereitstellen einer wässrigen Zubereitung enthaltend mindestens ein Kohlenhydrat;
(b) Konzentrieren der wässrigen Lösung aus Schritt (a) durch einen thermischen Verdampfungsschritt unter Erhalt eines wässrigen Konzentrats;
(c) Inkontaktbringen des wässrigen Konzentrats aus Schritt (b) mit einem Adsorptionsmitteln unter Erhalt eines entfärbten Konzentrats; sowie gegebenenfalls
(d) Kristallisieren des entfärbten Konzentrats aus Schritt (c),
wobei das Adsorptionsmittel ausgewählt ist aus
(i) Aktivkohlegranulat und
(ii) organischen synthetischen Harzen bzw. Ionenaustauschern.

## Beschreibung

### GEBIET DER ERFINDUNG

Die Erfindung befindet sich auf dem Gebiet der Lebensmitteltechnologie und betrifft ein Verfahren zur Entfärbung von wässrigen Kohlenhydratlösungen, speziell wässriger Allulose-Zubereitungen.

### TECHNOLOGISCHER HINTERGRUND

Unter die Zuckerersatzstoffe werden die Süßstoffe und die Zuckeralkohole gerechnet. Süßstoffe können auf natürlichem oder synthetischem Weg hergestellt werden. Sie zeichnen sich dadurch aus, dass sie keinen oder nur einen vernachlässigbar geringfügigen Nährwert besitzen, was sie für Menschen, die Diät halten, besonders interessant macht.

Süßstoff scheidet der Körper entweder ganz oder größtenteils unverändert aus. Sie finden sich in vielen Light- und Diätprodukten. Zuckeraustauschstoffe sind Kohlehydrate, die nur einen geringen Anstieg des Blutzucker- und Insulinspiegels verursachen. Ihre Süßkraft beträgt 40 bis 70 Prozent der Süßkraft normalen Haushaltszuckers. Der Anteil von Zuckerersatzstoffen in Lebensmitteln und Getränken hat in den letzten Jahren kontinuierlich zugenommen. Im Rahmen der vorliegenden Erfindung soll unter dem Begriff "niedrigkalorisch" ein Brennwert von nicht mehr als 10, vorzugsweise nicht mehr als 5 und insbesondere von 1 bis 2 kcal verstanden werden.

Die industriell bedeutsamsten Zuckerersatzstoffe sind Acesulfam K und Aspartam. Es handelt sich hierbei um synthetische Stoffe, die in einigen Studien als krebsgefährdend bezeichnet werden. Diese bisher nicht bestätigten Befunde stärken jedoch den allgemeinen Trend hin zu "natürlichen" Zuckerersatzstoffen.

In den letzten Jahren haben Stevioside breite Aufmerksamkeit erlangt, da die darin enthaltenen Einzelstoffe wie Rebaudiosid A eine bis zu 1000fach stärkere Süßkraft als Haushaltszucker besitzen und dabei nichtkalorisch sind. Nachteilig ist jedoch, dass Stevioside allgemein einen harten, metallischen Nachgeschmack aufweisen und sich daher bislang nicht als Zuckerersatzstoffe haben etablieren können.

Diese Lücke könnte geschlossen werden durch die so genannten "seltenen Zuckern" wie Tagatose, Cellobiose oder Allulose (die synonym auch als Psicose bezeichnet wird). Hierbei handelt es sich um Einfachzucker, die zwar nicht an die Süßkraft von Haushaltszucker herankommen, vom menschlichen Körper aber nicht verstoffwechselt werden. Sie gelten zudem als "natürlich", da sie in der Natur zu finden sind, wenn auch nur in geringen Mengen. Sie eignen sich daher insbesondere zum Teilaustausch in zuckerhaltigen Lebensmitteln.

### STAND DER TECHNIK

H. Itoh et al, Journal of Fermentation Bioengeneering, 80(1), 1995, S. 101-103 veröffentlicht die Herstellung von D-Psicose aus D-Fructose durch immobilisierte D-Tagatose 3-Epimerase.

N. Wagner et al in Organic Process Research Development 2012, 16, S. 323-330 bezieht sich auf praktische Aspekte des integrierten Betriebs von Biotransformation und simulierter Bewegungsbetttrennung (SMB) für die feinchemische Synthese. D-Psicose wird unter Verwendung der D-Tagatose Epimerase-katalysierten Epimerisierung aus D-Fructose hergestellt.

N. Wagner et al, in Chemical Engineering Science 137 (2015) S. 423-435 bezieht sich auf die modellbasierte Kostenoptimierung eines integrierten Prozesses zur enzymatischen Herstellung von Psicose bei erhöhten Temperaturen.

N. Wagner et al, in Angewandte Chemie Int. Ed. Engl. 2015, 54(14), S. 4182-6 offenbart eine trennungsintegrierte Kaskadenreaktion zur Überwindung thermodynamischer Einschränkungen bei der Seltenzucker-Synthese.

Bosshart et al, in Biotechnology Bioengineering 2016, 113(2), S. 349-58 bezieht sich auf die Produktion der seltenen Zucker D-Psicose und L-Tagatose durch zwei künstlich hergestellte D-Tagatose-Epimerasen.

N. Wagner, et al., in Journal of Chromatography A 2015, 1398, S. 47-56 offenbart ein Verfahren zur wirtschaftlichen Herstellung von d-Psicose durch simulierte Wanderbettchromatographie.

Gegenstand der WO 2018 087261 A1 (PFEIFER&LANGEN) ist ein Verfahren zur Synthese eines Einfachzuckers, vorzugsweise von D-Allulose aus einem Edukt, vorzugsweise von D-Fructose unter heterogener oder homogener Katalyse, die eine chemische und/oder enzymatische Katalyse beinhaltet, wobei die Synthese in mindestens zwei in Reihe geschalteten Reaktoren durchgeführt wird und das aus dem ersten Reaktor austretende Reaktionsprodukt vor dem Eintritt in den zweiten Reaktor einer chromatographischen Trennung unterzogen wird. Vorzugsweise wird die chromatographische Trennung in ein simuliertes Wanderbett integriert.

Aus der EP 2552241 B1 (CJ CHEIUEDANG) ist ein einstufiges Verfahren zur Konzentrierung von wässrigen Allulose-Lösungen bekannt, bei dem man eine übersättigte Zuckerlösung bei einer Temperatur von 60 bis 70 °C in der Lösung eindampft. Das Verfahren erfordert jedoch einen hohen Energieaufwand. Zudem sind hohe Verweilzeiten im Verdampfer erforderlich, was bei den anliegenden Temperaturen Anlass zu Maillard-Reaktionen gibt. Die resultierenden Produkte weisen daher häufig unerwünschte Verfärbungen auf, die eine Vermarktung erschweren.

Die EP 3564250 A2 (SAMYANG) empfiehlt zur Entfärbung von Verfärbungen von Allulose-Lösungen die Behandlung mit Aktivkohlepulver.

### AUFGABE DER ERFINDUNG

Infolge der Temperaturbelastung bei der Konzentrierung flüssiger Allulose-Zubereitungen, die als Intermediate anfallen und entweder als solche in den Handel gelangen ("Allulosesirup") oder einer Kristallisation unterworfen werden, kann es beispielsweise durch Maillard-Reaktion zu Verfärbungen der Kohlenhydrate kommen. Die Endprodukte weisen dann einen bräunlichen Farbton auf, was eine Vermarktung erschwert, weil der Verbraucher dies als Hinweis auf eine mindere Qualität versteht.

In der Praxis werden die flüssigen Allulose-Zubereitungen nach der Konzentrierung in der Regel einer Entfärbung unterworfen, bei der Aktivkohlepulver eingesetzt wird, das in den Sirup eingerührt und nach einer Verweilzeit abfiltriert wird. Nachteilig ist, dass der Filterkuchen praktisch nicht aufgearbeitet, sondern entsorgt werden muss und die Filtration viel Zeit in Anspruch nimmt und vergleichsweise technisch aufwendig ist. Beim Arbeiten mit Aktivkohlepulver fallen zudem Feinstäube an, was zusätzlichen Aufwand bei der Arbeitssicherung erfordert.

Die Aufgabe der vorliegenden Erfindung hat daher darin bestanden, aus dem Stand der Technik bekannte Entfärbungsverfahren zu verbessern, so dass in kürzeren Zeiten größere Mengen an farblich beeinträchtigten flüssigen Kohlenhydrat-Zubereitungen im Allgemeinen und Allulose-Zubereitungen im Besonderen, entfärbt werden können, wobei zum einen die Bildung von Feinstäuben vermieden wird und zum anderen das Entfärbungsmittel wieder aufgearbeitet werden kann. Da die verfärbten Produkte mitunter auch noch einen unerwünschten Nebengeschmack aufweisen, der sich durch Entfärbung mit Aktivkohlepulvern nicht entfernen lässt, hat eine weitere Aufgabe der Erfindung darin bestanden, auch die geschmacklichen Eigenschaften der Produkte zu verbessern.

### BESCHREIBUNG DER ERFINDUNG

Gegenstand der vorliegenden Erfindung ist ein Verfahren zur Herstellung farbloser Kohlenhydrate, umfassend oder bestehend aus den folgenden Schritten:
(a) Bereitstellen einer wässrigen Zubereitung enthaltend mindestens ein Kohlenhydrat;
(b) Konzentrieren der wässrigen Lösung aus Schritt (a) durch einen thermischen Verdampfungsschritt unter Erhalt eines wässrigen Konzentrats;
(c) Inkontaktbringen des wässrigen Konzentrats aus Schritt (b) mit einem Adsorptionsmittel unter Erhalt eines entfärbten Konzentrats; sowie gegebenenfalls
(d) Kristallisieren des entfärbten Konzentrats aus schritt (c),
wobei das Adsorptionsmittel ausgewählt ist aus
(i) Aktivkohlegranulat und
(ii) organischen synthetischen Harzen bzw. Ionenaustauschern.

Überraschenderweise wurde gefunden, dass Aktivkohle in granulierter Form sowie Adsorberharze nicht nur geeignetere Mittel darstellen, um Zuckerlösungen im Allgemeinen und Allulose-Verdampferkonzentrate im Besonderen in kurzen Zeiten mit hohem Durchsatz zu entfärben, sondern die resultierenden entfärbten Produkte zudem auch noch geschmackliche Vorteile aufweisen.

### KOHLENHYDRATE

Im Sinne der Erfindung kommen als Einsatzstoffe insbesondere niedrigkalorische Mono- und/oder Disaccharide mit vorzugsweise 6 bis 12 Kohlenstoffatomen in Frage. Beispiele umfassen vor allem Tagatose, Cellobiose, Xylose, Xylulose, Ribose, Ribulose, Sorbose, Allose, Altrose, Arabinose, Mannose, Gulose, Idose, Galactose, Talose, Nigerose, Kojibiose und Fructose. Besonders bevorzugt sind jedoch der Einsatz von Allulose bzw. Psicose und Konzentrierung von technischen Mischungen aus Allulose/Psicose und Fructose, die als solche bei der Epimerisierung von Fructose anfallen.

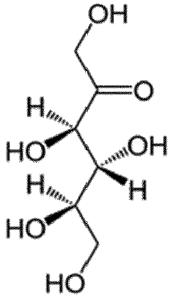

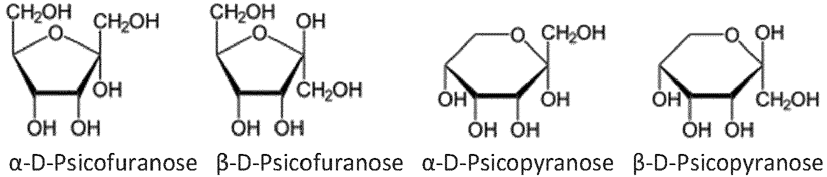

Die Herstellung von Psicose/Allulose durch Umlagerung von Fructose in Gegenwart einer Epimerase stellt freien Stand der Technik dar und geht auf Arbeiten der Universität Kagawa aus dem Jahre 1994 zurück. *Ken Izumori* entdeckte dabei, dass D-Tagatose 3-epimerase in der Lage ist, D-Fructose in D-Psicose umzulagern **(**Biosci. Biotechnol. Biochem. 58: 2168-2171**).** Die vorliegende Erfindung umfasst dabei auch jeweils Racemate, Stereoisomeren einzelner Kohlenhydrate sowie Mischungen verschiedener Kohlenhydrate, ohne dass im Folgenden jeweils einzeln noch einmal darauf hingewiesen wird.

### KONZENTRIERUNG

Bei dem Einsatzstoff gemäß Schritt (a) handelt es sich um eine beliebige wässrige Lösung eines der genannten Kohlenhydrate, darunter vor allem auch Zubereitungen, die als Sirup bezeichnet werden, wie auch Pulver, die in Wasser redispergiert worden sind.

Die wässrigen Lösungen im Allgemeinen und die oben beschriebenen technischen Mischungen im Besonderen, weisen typisch einen Feststoffgehalt von etwa 10 bis etwa 70 Gew.-% und insbesondere von etwa 20 bis etwa 50 Gew.-% auf. Allulose-Lösungen, die eingedampft werden sollen, weisen typisch einen Feststoffgehalt im Bereich von etwa 10 bis etwa 30 Gew.-% auf.

Der Verdampfungsprozess findet typisch entweder in einem oder in zwei bis drei in Reihe geschalteten Verdampfern statt. Bei den Bauteilen kann es sich Fallfilm- oder Plattenverdampfer handeln, die direkt oder indirekt mit Prozesswärme betrieben werden, wobei die Brüden in den Verdampfern oder Abscheidern über Kopf abdestilliert werden, während man die Sumpfprodukte - also die jeweiligen Konzentrate - von einer Stufe in die nächste leitet. Dabei wird die Trockensubstanz in der Regel auf etwa 55 bis etwa 90 Gew.-% angehoben. Ausgehend von einer Allulose-Lösung mit einem Trockensubstanzgehalt von typisch etwa 10 bis 25 Gew.-% oder alternativ etwa 50 bis etwa 70 Gew.-% wird durch Verdampfung dann ein Konzentrat mit einer typischen Trockensubstanz von etwa 80 bis etwa 90 Gew.-% erhalten.

Die Verdampfung wird in der Regel bei einer Temperatur von etwa 50 bis etwa 75 °C durchgeführt. Wird der Verfahrensschritt nicht einstufig, sondern in zwei oder drei Stufen durchgeführt, hat es sich aus energetischen Gründen als vorteilhaft erwiesen, bei der Temperaturführung den Bereich zwischen 59 und 71 °C auszulassen, also in den Verdampfern in Temperaturbereichen darüber und darunter zu arbeiten.

### ENTFÄRBUNG

Zur Entfärbung werden die Verdampferkonzentrate mit den Entfärbungsmitteln, also entweder den Aktivkohlegranulaten oder den organischen synthetischen Harzen und Ionenaustauschern, über eine ausreichende Zeitdauer in Kontakt gebracht.

Das Aktivkohlegranulat hat dabei vorzugsweise eine Körnung bei der 90 % der Teilchen einen Durchmesser von etwa 0,5 bis etwa 1 mm aufweisen. Besonders bevorzugt ist ein Granulat der Firma Cabot mit einem durchschnittlichen Partikeldurchmesser von etwa 0,65 mm, das unter der Bezeichnung Norit GAC 1240 erhältlich ist.

Die bevorzugten Adsorptionsmittel stellen makroporöse Harze dar, wie sie im Folgenden exemplarisch aufgeführt sind: Lewatit A 365, Lewatit A 8071, Lewatit A 8072, Lewatit A 8072 PLUS, Lewatit A 8073, Lewatit AF 5, Lewatit S 1567, Lewatit S 1568, Lewatit S 1668, Lewatit S 2328, Lewatit S 2568, Lewatit S 2568 H, Lewatit S 4228, Lewatit S 4268, Lewatit S 4328, Lewatit S 4468, Lewatit S 4528, Lewatit S 5128, Lewatit S 5221, Lewatit S 5228, Lewatit S 5328, Lewatit S 5528, Lewatit S 6268, Lewatit S 6368, Lewatit S 6368 A, Lewatit S 6368 A OH, Lewatit S 6368 A SO4, Lewatit S 6368 Sulfat, Lewatit S 7468, Lewatit S 7968, Lewatit S 8107, Lewatit S 8223, Lewatit S 8227, Lewatit S 8227 Ca, Lewatit S 8227 Mg, Lewatit S 8229, Lewatit S 8229 Plus / Ag, Lewatit S 8229 PLUS X, Lewatit S 8528, Lewatit S 9167, Dowex HCR-S/S, Dowex HCR-S/S FF, Dowex HCR-W2, Dowex-HGR-NG, Dowex Marathon C 10, Dowex Monosphere C 350, Dowex Monosphere C 400, Dowex Marathon C , Dowex Marathon MSC, Dowex Marathon 1200, Dowex Marathon 1300 H, Dowex 88, Dowex 88 MB, Dowex Monosphere 88 MB, Dowex Monosphere C 600 B, Dowex 66, Dowex Monosphere 66, Dowex 22, Dowex Optipore SD 2, Dowex Optipore L493, Dowex Optipore V493, Dowex Optipore V502, XUS 43565.01, Dowex Optipore V323, Dowex N 406, Dowex Marathon A, Dowex Marathon A LB, Dowex Marathon A2, Dowex Marathon MSA, Dowex Marathon 11, Dowex Marathon MSA -, Dowex NSR-1, Dowex-PSR-2, Dowex Marathon 4200 Cl, Treverlite IXC100, Treverlite IXC110, Treverlite IXC200, Treverlite IXC201, Treverlite IXC210, Treverlite IXC230, Treverlite IXC330, Treverlite IXA300, Treverlite IXA310, Treverlite IXA510, Treverlite IXA600, Treverlite IXA610, Treverlite IXA620, Treverlite IXA710, Treverlite CHE710, Treverlite CHE720, Treverlite CHE730, Treverlite XS100200, Treverlite XS103500, Treverlite XS106500, Treverlite XS122400. Bei den entsprechenden Entfärberharzen handelt es sich um kommerzielle Produkte, die von den Herstellern Lanxess, Dow und Chemra bezogen werden können. Besonders gute Ergebnisse wurden dabei mit den Ionenaustauschern erzielt, die als Matrix Styrol-Divinylbenzol-Copolymere, quernetzte Polystyrene, quervernetztes Phenol-Formaldehyd-Kondensat oder Mischungen von diesen aufweisen. Als kationische Gruppen kommen vor allem tertiäre Amine und quaternäre Amine, als anionische Gruppen Sulfonsäuren und Sulfonate in Betracht.

Die Entfärberharze bzw. das Aktivkohlegranulat können in die Verdampferkonzentrate eingerührt und nach einer Verweilzeit von einigen Minuten bis zu einer Stunde abfiltriert werden. Es hat sich aber als vorteilhaft erwiesen, die Entfärbung kontinuierlich über Adsorptionssäulen durchzuführen, die mit dem Granulat oder den synthetischen Harzen gefüllt sind. Über Durchsatz und Länge der Säule lässt sich die Verweilzeit so steuern, dass am Ende der Säule(n) schließlich eine Lösung mit gewünschter Farbreduktion austritt. Es empfiehlt sich zwei Reihen Säulen parallel zu fahren, so dass eine im Wechsel jeweils gereinigt werden kann.

Besonders bevorzugt ist eine Ausführungsform, bei der man zwei oder mehr Säulen in Reihe schaltet und mit unterschiedlichen Adsorptionsmitteln füllt, insbesondere ein Adsorberharz in der ersten und ein Aktivkohlegranulat in der zweiten Säule.

Gegenüber Aktivkohlepulver weist das Granulat zwar den Nachteil auf, dass es eine geringere spezifische Oberfläche besitzt, dies wird jedoch dadurch mehr als ausgeglichen, dass das Granulat in Säulen angeordnet werden kann, die von den Verdampferkonzentraten durchströmt werden können. Die Granulierung der Aktivkohle sowie die Form der Harzpartikel führt gegenüber dem Einsatz von pulverförmigen Agenzien zu einem geringeren Druckverlust beim Durchströmen einer gepackten Säule und daher zu einem niedrigeren Energieverbrauch. Zudem hat sich der Einsatz von gepackten Säulen dadurch als vorteilhaft erwiesen, dass das Harzmaterial beziehungsweise die Aktivkohlegranulate durch die Säulen zurückgehalten und nicht mehr in einem zusätzlichen Schritt von der zu entfärbenden Lösung abgetrennt werden müssen. Werden Harze eingesetzt, hat dies weiter den Vorteil, dass diese in einfacher Weise chemisch regeneriert werden können. Die Aktivkohle muss hingegen nach dem Erreichen der Entfärbungskapazität aufwändig ausgebrannt werden. Sowohl beim Einsatz von Aktivkohlegranulat als auch synthetischen Harzen fällt zudem kein Feinstaub an.

### KRISTALLISATION

Das entfärbte Verdampferkonzentrat ("Sirup") kann als solches kommerzialisiert oder zur Kristallisation eingesetzt werden. Typischerweise hat das Konzentrat einen Feststoffgehalt von 55 bis 90 Gew.-% (im Fall von Allulose-Konzentraten von etwa 75 bis etwa 90 Gew.-%) und wird anschließend im übersättigten Bereich, nahe des Sättigungspunkts, mit Impfkristallen versetzt. Die Übersättigung wird anschließend durch Verdampfung oder langsames Abkühlen (0,1-1K/h) aufrechterhalten bis sich ein Kristallgehalt von etwa 30 bis etwa 50 Gew.-% eingestellt hat.

### BEISPIEL 1

### Entfärbung von Allulose-Lösungen

Es wurden 15 kg Stammlösung mit einem hohen Farbgehalt hergestellt. Hierzu wurde eine 60 Gew.-%ige Allulose-Lösung mit 1 Gew.-% Dikaliumhydrogenphosphat für 6 Stunden bei 90 °C in einem Rührreaktor behandelt. Die Stammlösung wies einen ICUMSA Farbwert (bestimmt nach GS 9(1/2/3-8 (2011) von ca. 9.000 IE auf wurde anschließend durch Zugabe von Wasser auf einen Farbwert von 500 bzw. 1370 IE eingestellt.

Zwei beheizbare Glaskolonnen jeweils mit einer Länge von 100 cm und einem Durchmesser von 5 cm wurden mit einer Schüttung verschiedener Adsorptionsmitteln versehen, in Reihe geschaltet und mit einem Durchsatz von 25 L/h mit dem Feed beaufschlagt. Der Farbwert wurde jeweils nach dem Verlassen der Kolonnen photometrisch bestimmt. Die Änderung der Farbwerte gegen die Standzeit des Adsorptionsmittels sind in **Tabelle 1** wiedergegeben:

**Tabelle 1A**

| Entfärbung im System Lewatit S 5221/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 500 | 500 | 500 | 500 | 50 | 110 | 150 | 175 |
| Farbzahl Produkt [IE] | 50 | 110 | 150 | 175 | 25 | 65 | 90 | 95 |

**Tabelle 1B**

| Entfärbung im System Lewatit S 5221/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 1.370 | 1.370 | 1.370 | 1.370 | 200 | 240 | 290 | 380 |
| Farbzahl Produkt [IE] | 200 | 240 | 290 | 380 | 100 | 110 | 140 | 170 |

Nach Verlassen der ersten Kolonne wurde in beiden Fällen ein Produkt erhalten, dessen Farbzahl auf 10 % des Ausgangswertes vermindert worden war. Über eine Standzeit von 8 Stunden stieg der Wert bis auf etwa 20 % des Ausgangsfarbwertes an. Mit der zweiten Kolonne ("Polisher") konnte der Farbwert jeweils noch einmal um 50 % gesenkt werden.

### BEISPIEL 2

### Entfärbung von Allulose-Lösungen

Es wurden 15 kg Stammlösung mit einem hohen Farbgehalt hergestellt. Hierzu wurde eine 60 Gew.-%ige Fructose-Lösung mit 1 Gew.-% Dikaliumhydrogenphosphat für 6 Stunden bei 90 °C in einem Rührreaktor behandelt. Die Stammlösung wies einen ICUMSA Farbwert (bestimmt nach GS 9(1/2/3-8 (2011) von ca. 8600 IE auf wurde anschließend durch Zugabe von Wasser auf einen Farbwert von 500 bzw. 1370 IE eingestellt.

Zwei beheizbare Glaskolonnen jeweils mit einer Länge von 100 cm und einem Durchmesser von 5 cm wurden mit einer Schüttung verschiedener Adsorptionsmitteln versehen, in Reihe geschaltet und mit einem Durchsatz von 25 L/h mit dem Feed beaufschlagt. Der Farbwert wurde jeweils nach dem Verlassen der Kolonnen photometrisch bestimmt. Die Änderung der Farbwerte gegen die Standzeit des Adsorptionsmittels sind in **Tabelle 2** wiedergegeben:

**Tabelle 2A**

| Entfärbung im System Dowex Monosphere 88 MB/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 500 | 500 | 500 | 500 | 60 | 120 | 140 | 180 |
| Farbzahl Produkt [IE] | 60 | 120 | 140 | 180 | 30 | 70 | 85 | 90 |

**Tabelle 2B**

| Entfärbung im System Dowex Monosphere 88 MB/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 1.370 | 1.370 | 1.370 | 1.370 | 220 | 250 | 300 | 400 |
| Farbzahl Produkt [IE] | 220 | 250 | 300 | 400 | 110 | 120 | 150 | 160 |

Nach Verlassen der ersten Kolonne wurde auch hier in beiden Fällen ein Produkt erhalten, dessen Farbzahl auf 10 % des Ausgangswertes vermindert worden war. Über eine Standzeit von 8 Stunden stieg der Wert bis auf etwa 20 % des Ausgangsfarbwertes an. Mit der zweiten Kolonne ("Polisher") konnte der Farbwert jeweils noch einmal um 50 % gesenkt werden.

## Patentansprüche

1. Verfahren zur Herstellung farbloser Kohlenhydrate, umfassend oder bestehend aus den folgenden Schritten:
(a) Bereitstellen einer wässrigen Zubereitung enthaltend mindestens ein Kohlenhydrat;
(b) Konzentrieren der wässrigen Lösung aus Schritt (a) durch einen thermischen Verdampfungsschritt unter Erhalt eines wässrigen Konzentrats;
(c) Inkontaktbringen des wässrigen Konzentrats aus Schritt (b) mit einem Adsorptionsmittel unter Erhalt eines entfärbten Konzentrats; sowie gegebenenfalls
(d) Kristallisieren des entfärbten Konzentrats aus Schritt (c),
wobei das Adsorptionsmittel ausgewählt ist aus
(i) Aktivkohlegranulat und
(ii) organischen synthetischen Harzen bzw. Ionenaustauschern.

2. Verfahren nach Anspruch 1, **dadurch gekennzeichnet, dass** man wässrige Zubereitungen von Kohlenhydraten einsetzt, die ausgewählt sind aus der Gruppe, die gebildet wird von Allulose, Tagatose, Cellobiose, Xylose, Xylulose, Ribose, Ribulose, Sorbose, Allose, Altrose, Arabinose, Mannose, Gulose, Idose, Galactose, Talose, Nigerose, Kojibiose und Fructose.

3. Verfahren nach den Ansprüchen 1 und/oder 2, **dadurch gekennzeichnet, dass** die wässrigen Zubereitungen einen Trockensubstanzgehalt von etwa 10 bis etwa 70 Gew.-% aufweisen.

4. Verfahren nach Anspruch 3, **dadurch gekennzeichnet, dass** die wässrige Zubereitung eine Allulose-Lösung mit einem Trockensubstanzgehalt von etwa 20 bis etwa 50 Gew.-% darstellt.

5. Verfahren nach mindestens einem der Ansprüche 1 bis 4, **dadurch gekennzeichnet, dass** man die wässrigen Zubereitungen durch Verdampfung auf einen Trockensubstanzgehalt von etwa 55 bis etwa 90 Gew.-% einstellt.

6. Verfahren nach Anspruch 5, **dadurch gekennzeichnet, dass** man eine Allulose-Lösung mit einem Trockensubstanzgehalt von etwa 50 bis etwa 70 Gew.-% durch Verdampfung auf einen Trockensubstanzgehalt von etwa 80 bis etwa 90 Gew.-% einstellt.

7. Verfahren nach mindestens einem der Ansprüche 1 bis 6, **dadurch gekennzeichnet, dass** man die Verdampfung in einer, zwei oder drei Stufen durchführt.

8. Verfahren nach mindestens einem der Ansprüche 1 bis 7, **dadurch gekennzeichnet, dass** man die Verdampfung bei einer Temperatur von etwa 50 bis etwa 75 °C durchführt.

9. Verfahren nach mindestens einem der Ansprüche 1 bis 8, **dadurch gekennzeichnet, dass** man die Verdampfung in zwei oder drei Stufen durchführt und bei der Temperaturführung den Bereich zwischen 59 und 71 °C auslässt.

10. Verfahren nach mindestens einem der Ansprüche 1 bis 9, **dadurch gekennzeichnet, dass** man die Entfärbung in einer Adsorptionssäule durchführt.

11. Verfahren nach Anspruch 10, **dadurch gekennzeichnet, dass** man die Entfärbung kontinuierlich in zwei Adsorptionssäulen durchführt, die im Wechsel betrieben werden.

12. Verfahren nach mindestens einem der Ansprüche 1 bis 11, **dadurch gekennzeichnet, dass** man Aktivkohlegranulat einsetzt, bei dem 90 % aller Teilchen einen Durchmesser im Bereich von 0,1 bis 1 mm aufweist.

13. Verfahren nach mindestens einem der Ansprüche 1 bis 12, **dadurch gekennzeichnet, dass** man organische synthetische Harze bzw. Ionenaustauscher einsetzt, die
(i) als Matrix Styrol-Divinylbenzol-Copolymere, quernetzte Polystyrene, quervernetztes Phenol-Formaldehyd-Kondensat oder Mischungen von diesen, und/oder
(ii) als kationische Gruppen tertiäre Amine und/oder quaternäre Amine, und/oder
(iii) als anionische Gruppen Sulfonsäuren und/oder Sulfonate aufweisen.

14. Verfahren nach mindestens einem der Ansprüche 10 bis 13, **dadurch gekennzeichnet, dass** man mindestens zwei Adsorptionssäulen in Reihe schaltet.

15. Verfahren nach Anspruch 14, **dadurch gekennzeichnet, dass** man zwei Adsorptionssäulen in Reihe schaltet und die erste mit einem organischen synthetischen Harz und die andere mit Aktivkohlegranulat füllt.
